# FASCICULE DE BREVET EUROPEEN

(11) **EP 2 882 290 B1**
(45) Date de publication et mention de la délivrance du brevet: **14.08.2019**
(21) Numéro de dépôt: 13724530.4
(22) Date de dépôt: 03.05.2013
(51) Int. Cl.: A01N 1/02, G01N 33/50, C12Q 1/00, C12N 5/071

(54) **SYSTEME PERMETTANT LA MAINTENANCE EN SURVIE ET LE TRANSPORT DE BIOPSIES DE PEAU ET SES APPLICATIONS**
SYSTEM ZUR LEBENDHALTUNG UND ZUM TRANSPORT VON HAUTBIOPSIEN UND ANWENDUNGEN DIESES SYSTEMS
SYSTEM FOR KEEPING ALIVE AND TRANSPORTING SKIN BIOPSIES AND APPLICATIONS OF SAID SYSTEM

(30) Priorité: 03.05.2012 FR 1254091
(43) Date de publication de la demande: 17.06.2015
(73) Titulaire: Genoskin, 31400 Toulouse (FR)
(72) Inventeur: DESCARGUES, Pascal, F-31500 Toulouse (FR)
(74) Mandataire: Barbot, Willy
(86) Numéro de dépôt international: PCT/EP2013/059215
(87) Numéro de publication internationale: WO 2013/164436

(56) Documents cités:
- EP-A2- 0 702 081
- EP-A2- 2 019 316
- WO-A1-2012/059703
- WO-A2-2004/022696
- US-A1- 2001 049 140
- US-A1- 2003 040 113
- US-A1- 2011 045 477
- JACOBS J J L ET AL: "METHYL GREEN-YPRONINE STAINING OF PORCINE ORGANOTYPIC SKIN EXPLANT CULTURES: AN ALTERNATIVE MODEL FOR SCREENING FOR SKIN IRRITANTS", ATLA. ALTERNATIVES TO LABORATORY ANIMALS, LONDON, GB, vol. 28, 1 janvier 2000 (2000-01-01), pages 279-292, XP009038188, ISSN: 0261-1929
- LEBONVALLET N ET AL: "The evolution and use of skin explants: potential and limitations for dermatological research", EUROPEAN JOURNAL OF DERMATOLOGY, JOHN LIBBEY EUROTEXT, FR, vol. 20, no. 6, 1 novembre 2010 (2010-11-01), pages 1-14, XP009141240, ISSN: 1167-1122 [extrait le 2010-09-07]
- KISHI K ET AL: "Treatment of giant congenital melanocytic nevi with enzymatically separated epidermal sheet grafting", JOURNAL OF PLASTIC, RECONSTRUCTIVE AND AESTHETIC SURGERY, CHURCHILL LIVINGSTONE, GB, vol. 63, no. 6, 1 juin 2010 (2010-06-01), pages 914-920, XP002626328, ISSN: 1748-6815, DOI: 10.1016/J.BJPS.2009.03.010 [extrait le 2009-04-26]

## Description

### DOMAINE DE L'INVENTION

La présente invention concerne un procédé *in vitro* ou *ex vivo* pour la conservation et/ou la maintenance en survie de biopsies de peau de mammifère, de préférence humaine, permettant son transport et, le cas échéant sa culture. La présente invention divulgue également une biopsie de peau ainsi conservée par un tel procédé et son utilisation comme modèle notamment dans un kit pour le criblage ou la sélection de composés cosmétiques ou thérapeutiques.

### ART ANTERIEUR

La peau est constituée par la superposition de l'épiderme et du derme. L'épiderme est un épithélium pavimenteux pluristratifié et squameux. Il forme une barrière qui résiste aux ravages de la dessiccation, ainsi qu'aux agressions mécaniques, chimiques et microbiennes. Le type cellulaire principal constituant l'épiderme est le kératinocyte. Ce tissu comprend également d'autres populations cellulaires telles que les mélanocytes, les cellules de Langerhans, et les cellules de Merkel (voir annexe). L'épiderme est conventionnellement sous divisé en quatre strates distinctes comprenant des couches internes vers les couches les plus superficielles : la couche basale (une couche), la couche épineuse (4-15 couches), la couche granuleuse (1-3 couches) et la couche cornée (5-10 couches). L'épiderme repose sur le derme grâce une membrane basale constituée entre autres de molécules de collagène. Le derme contient des réseaux vasculaires et nerveux très denses ainsi que les annexes épidermiques, structures kératinisées prologeant l'épiderme et incluant les follicules pileux, les glandes sébacées, et les glandes sudoripares. La peau repose sur un tissus sous-cutanée, appelé hypoderme, principalement constitué de cellules graisseuses et participant, entre autres, à l'élasticité de la peau et à la thermorégulation de l'organisme.

Les systèmes de culture *in vitro* ou *ex vivo* de peau sont depuis longtemps développés pour la recherche académique ou appliquée (Lebonvallet et al. « The evolution and use of skin explants: potential and limitations for dermatological research." Eur J Dermatol 2010; 20 (6): 671-84). Désormais, le banissement de l'utilisation des animaux pour le développement des produits cosmétiques en Europe (Pauwel M and Rogiers V. « Human health safety evaluation of cosmetics in the EU: a legally imposed challenge to science. » Toxicol Appl Pharmacol. 2010 Mar 1;243(2):260-74), ainsi que l'importance prise par la règle des 3Rs (Réduire, Remplacer, Diminuer) concernant l'expérimentation animale lors du développement pharmaceutique (Wells DJ. « Animal welfare and the 3Rs in European biomedical research. » Ann N Y Acad Sci. 2011 Dec; 1245:14-6), confèrent aux systèmes in vitro ou ex vivo de culture de peau, en particulier ceux permettant la culture de peau humaine, une importance stratégique pour les industries cosmétiques, chimiques et pharmaceutiques. Les systèmes de culture *in vitro* ou *ex vivo* de peau sont des modèles de choix pour l'étude de la biologie cutanée. Ils possèdent en effet tous les types cellulaires de la peau, organisés dans une structure en trois dimensions (3D). Ils reflètent directement les paramètres d'individus, comme l'âge, le sexe, l'état pathologique de la peau, ou l'exposition au soleil (Lebonvallet *et al.,* 2010).

Si les systèmes ou dispositifs de culture *in vitro* ou *ex vivo* de peau sont des modèles de choix pour la recherche, les systèmes ou dispositifs existants sont mal adaptés à leur transport et maintenance en survie.

Plusieurs systèmes ou dispositifs de culture, plus ou moins perfectionnés existent (Lebonvallet *et al.,* 2010), cependant ces systèmes ou dispositifs, sont en général fragiles et ne permettent pas leur expédition par route ou par les airs, sans que cela puisse affecter leur intégrité. En effet, dans ces systèmes ou dispositifs, la biopsie de peau n'est pas fermement maintenue par un support physique. Elle peut être laissée flottante dans une solution de milieu de culture ou déposé sur un insert à membrane poreuse ou sur une grille en acier inoxydable. L'avantage des deux derniers procédés est de permettre de maintenir la partie superficielle de l'épiderme, la couche cornée, directement en contact avec l'air atmosphérique. Ceci permet l'étude de l'application topique de substances à la surface de la peau.

On peut également citer ici les documents brevets publiés sous les numéros EP 2 019 316, US2011/045477, ou WO2004/092354 qui décrivent des systèmes de culture *in vitro* ou *ex vivo* de peau, mais qui ne mentionnent aucun support ou dispositif pour le maintien et le transport des biopsies de peau.

Plus récemment, un système de chambre en acier inoxydable a été développé pour maintenir fermement de larges biopsies de peau et permettre leur culture in vitro ou ex vivo jusqu'à 4 semaines (Lars Steinstraesser et al. « A Human Full-Skin Culture System for Interventional Studies." Eplasty. 2009;9:e5.). Bien qu'intéressant, ce système est peu adapté à la culture cellulaire du fait même de ses dimensions et sa composition (acier inoxydable). De plus, ce système est basé sur une mise en tension importante de la peau, qui ne reflète pas les conditions physiologiques rencontrées pour cet organe *in vivo.* Comme art antérieur le plus proche de la présente invention, on peut également citer EP 0 702 081 qui divulgue un procédé *ex vivo* pour la maintenance d'un fragment de peau artificielle comprenant deux types d'éponges de collagène de densité différente, inoculées avec des fibroblastes et des kératinocytes, enserrées dans une matrice constituée de gélatine. La publication de Jacobs et al. (« Methyl green-pyronine staining of porcins organotypic skin explant cultures : an alternative model for screening for skin irritants », ATLA, 28,279-92, 2000) décrit l'utilisation de biopsies de peau de porc placées dans du milieu de culture, traitées avec du produit irritant puis incluses dans une matrice solide, avant d'être congelées puis colorées. US 2003/040113 décrit un support multicouche pour la culture cellulaire, dans lequel le support cellulaire est ensemencé avec des cellules vivantes suspendues dans un gel. WO 2004/022696 décrit un support de fibrine, comprenant du fibrinogène et de la thrombine, utilisé pour la culture de cellules, notamment de keratinocytes. Enfin, WO 2012/059703, déposé avant la présente demande et publié le 10 mai 2012, soit après le dépôt de la présente demande, décrit un procédé pour la conservation et/ou la maintenance en survie de l'épiderme.

Ces documents ne divulguent ni ne suggèrent un procédé pour la maintenance en survie et le transport d'un fragment ou d'une biopsie de peau déposé dans une matrice liquide apte à se solidifier, et donc maintenu fermement par la matrice mais également nourri, l'ensemble étant contenu dans un insert dont le fond est constitué d'une membrane poreuse.

En ce qui concerne les biopsies de peau, l'étude de certains milieux de culture utilisés pour maintenir *in vitro* ou *ex vivo* ces biopsies de peau ont mis en évidence l'effet délétaire de la présence de sérum sur la structure de la peau, alors que la présence d'ions calcium à une concentration de 1,4 mM est importante pour la cohésion du tissue, via la stimulation de la production de matrice extracellulaire et de la différenciation épidermique (Lebonvallet *et al.,* 2010).

Depuis de nombreuses années, on recherche à mettre au point de nouveaux procédés visant à pouvoir conserver et/ou maintenir en survie des biopsies de peau de mammifère, de préférence humaine ou de porc. Ceci d'autant plus si ces procédés permettent le transport et la culture de ces biopsies.

Le développement de tels modèles est en effet très important en particulier pour la recherche dermatologique et pour les études nécessaires à l'élaboration de produits pharmaceutiques et/ou cosmétiques.

Ainsi, il serait souhaitable de pouvoir disposer d'un système, dispositif et/ou procédé permettant d'obtenir de telles biopsies de peau et suffisamment robustes pour permettre leur transport (que ce soit par voie aérienne et/ou terrestre). L'obtention d'un tel modèle permettrait de préserver l'organisation 3D et les fonctions de la peau à partir d'une biopsie, et ceci avantageusement par rapport aux cultures de peau reconstruite habituellement longues à développer et nécessitant des techniques complexes (les biopsies étant plus faciles à obtenir et ayant un plus grand potentiel à l'égard de la recherche dermatologique, en particulier pour les études portant sur la matrice extracellulaire, structure 3D ou les interactions entre les différents types de cellules cutanées).

De tels modèles auraient comme avantages de pouvoir permettre la réalisation d'études nécessaires à une meilleure compréhension du rôle de la peau, notamment de son épiderme et du derme, tant dans le domaine mécanique que dans le domaine physiologique. De telles biopsies solidement maintenues grâce à un tel système, dispositif et/ou procédé robuste, permettraient également le prélèvement de couches cellulaires de la couche cornée de l'épiderme par des détachements superficiels successifs réalisés à l'aide d'un ruban autocollant (tape stripping). De telles biopsies ainsi préservées et transportables peuvent également être utilisés comme modèles de choix, comparés aux modèles de peau reconstruite et leurs inconvénients (voir Lebonvallet *et al.,* 2010) pour la prédiction par des tests *in vitro* ou *ex vivo,* de l'activité de principe actifs cosmétiques et/ou pharmaceutiques ou encore des effets secondaires de composé topiques.

Ceci est justement l'objet de la présente invention.

### DESCRIPTION DETAILLEE DE L'INVENTION

De façon surprenante, la demanderesse a mis en évidence que la mise en oeuvre du procédé de l'invention et du dispositif qui en découle tel que décrit ci-après permettent d'obtenir des biopsies de peau aptes à être conservées et/ou transportées, et pouvant servir, le cas échéant, de modèle d'expérimentation.

L'invention a donc pour objet un procédé *ex vivo* ou *in vitro* pour la maintenance en survie *in vitro* ou *ex vivo* et le transport de biopsie de peau, ladite biopsie de peau comprenant au moins l'épiderme, le derme et les annexes épidermiques, ladite biopsie ayant été préalablement prélevée chez un mammifère, ladite méthode comprenant les étapes suivantes :
a) Le dépôt de ladite biopsie de peau sur une matrice liquide apte à pouvoir se solidifier sous l'action d'une augmentation ou d'une diminution de la température et/ou par l'addition d'un composé ou d'une composition spécifique de manière à ce que la partie superficielle de ladite biopsie correspondant à la totalité de l'épiderme reste émergée alors que le derme sous-jacent à cet épiderme est immergé totalement, ladite matrice liquide apte à pouvoir se solidifier étant choisie parmi le plasma sanguin ou une solution dérivée de plasma sanguin dilué en tampon physiologique à au maximum 10%, une solution de fibrinogène, une solution de collagène, de la gélatine, les gels polymériques synthétiques, les gels naturels d'agarose ou d'agar-agar à faible/bas points de fusion, les gels d'amidon ou de polysaccharides, ou un de leurs mélanges,
   ladite matrice liquide apte à pouvoir se solidifier étant elle-même contenue dans un insert dont le fond est constitué d'une membrane poreuse, et
   ledit insert étant disposé dans un container ou puits; et
b) La solidification de ladite matrice liquide apte à pouvoir se solidifier induite par une augmentation ou une diminution de la température et/ou par l'addition d'un composé ou d'une composition spécifique, cette solidification ayant pour effet d'emprisonner la partie immergée de ladite biopsie de peau dans ladite matrice ainsi solidifiée, et de faire adhérer ladite matrice solidifiée aux parois latérales et à ladite membrane poreuse de l'insert, et ladite partie superficielle épidermique de la biopsie, restant émergée, étant en contact avec l'air atmosphérique ou sous une atmosphère contrôlée comprenant en partie de l'air.

De préférence, ledit insert est disposé dans un container ou un puits vide (type cupule de microplaque ou plaque pouvant servir à la culture cellulaire, de préférence à fond plat).

Selon un mode de réalisation particulier, un procédé selon l'invention comprend, préalablement au dépôt de la biopsie de peau sur une matrice liquide apte à pouvoir se solidifier sous l'action d'une augmentation ou d'une diminution de la température et/ou par l'addition d'un composé ou d'une composition spécifique, une étape de fixation à la surface épidermique de la biopsie de peau d'un anneau constitué par un matériau hydrophobe, le diamètre extérieur dudit anneau étant supérieur au diamètre de la surface épidermique de la biopsie, le diamètre intérieur dudit anneau étant inférieur au diamètre de la surface épidermique de la biopsie.

Plus précisément, la fixation de l'anneau est réalisée préalablement à l'étape a) de dépôt du de la biopsie, et préalablement à l'étape i) de flottaison de la biopsie.

De préférence, le matériau hydrophobe de l'anneau est un matériau non toxique pour la peau, il peut être un polymère de paraffine, de type Parafilm ® (Sigma), ou un polymère de silicone. Selon un mode particulier, l'anneau est préparé à partir d'un film de matière hydrophobe, par la perforation de ce film selon les dimensions désirées. L'épaisseur de l'anneau est de préférence comprise entre 0,1 mm et 2 mm, de préférence entre 0,1 et 1 mm, plus préférentiellement entre 0,1 mm et 1 mm, plus préférentiellement entre 0,1 et 0,5 mm, et encore plus préférentiellement entre 0,12 et 0,2 mm.

Ledit anneau peut être constitué par un matériau opaque ou translucide. Selon un mode de réalisation particulier, l'anneau est constitué par un matériau opaque.

Selon un mode de réalisation plus particulier, ledit anneau est fixé à la surface épidermique de la biopsie à l'aide de colle, de préférence ajoutée à la surface inférieure de l'anneau. Ladite colle peut être choisie parmi tout type de matériau non toxique pour la peau et ayant pour effet l'adhésion de l'anneau à la peau, ce matériau pouvant être du silicone. De préférence, ladite colle est hydrophobe.

De préférence, après l'étape b), ledit container ou puits, est recouvert par un couvercle et, le cas échéant rendu étanche, et, le cas échéant, enveloppé dans une enveloppe protectrice apte au transport.

Une matrice liquide apte à pouvoir se solidifier est une solution liquide comprenant au moins un composé ou une composition spécifique dont la concentration dans ladite solution liquide est telle que, lors de la mise en oeuvre de conditions appropriées, notamment des conditions particulières de température, la solution liquide prend une consistance de type solide ou gélifiée. Ledit composé ou une composition spécifique peut être d'origine animale, végétale ou synthétique, sa nature et sa concentration sont déterminés en fonction des caractéristiques physico-chimiques souhaitées de la matrice lorsqu'elle est solidifiée, notamment la souplesse et la résistance de la matrice.

Selon un mode de réalisation du procédé selon l'invention, à l'étape a), ladite matrice liquide apte à pouvoir se solidifier est choisie parmi toute solution liquide, de préférence nutritive, capable de se solidifier ou de se gélifier dans des conditions particulières compatibles avec la survie et la culture de cellules de peau qui composent ladite biopsie.

La présente invention divulgue également un procédé *ex vivo* ou *in vitro* de conservation ou de maintenance en survie d'une biopsie de peau, ladite biopsie étant apte à être transportée, ladite biopsie ayant été préalablement prélevée chez un mammifère, ou obtenue à partir d'une collection ou banque d'échantillon de peau, ou encore issu d'une peau en culture, caractérisé en ce que ledit procédé comprend les étapes suivantes :
i) la flottaison d'une biopsie de peau cylindrique dans une matrice liquide choisie parmi une solution dérivée de plasma sanguin, solution de fibrinogène ou de collagène, ou d'une solution d'agar-agar ou d'agarose à bas point de fusion, ou un mélange de ces solutions, cette matrice liquide étant contenue dans ledit insert, cette flottaison laissant la surface épidermique de la biopsie émergée, tandis que le derme est immergé ;
ii) l'induction rapide de la solidification de ladite entourant la biopsie de peau ; et,
iii) La culture *in vitro* ou *ex vivo,* ou la maintenance en survie de la biopsie de peau dans ladite matrice ainsi solidifiée.

De préférence, ladite matrice liquide ou solidifiée ne contient aucun facteur de croissance, ni sérum.

A l'étape i), la flottaison d'une biopsie de peau cylindrique est effectuée dans une matrice liquide comprenant une première solution choisie parmi une solution dérivée de plasma sanguin, une solution de fibrinogène ou une solution de collagène, et une deuxième solution d'agar-agar ou d'agarose à bas point de fusion. Lorsque la matrice liquide contient une solution d'agar-agar ou d'agarose à bas point de fusion, cette deuxième solution est préalablement chauffée pendant une durée et à une température suffisante pour être liquide et pour rester liquide à environ 37°C pendant le temps suffisant pour être mélangée à la première solution dans ledit insert et jusqu'au dépôt de ladite biopsie de peau.

De préférence, ladite deuxième solution est préalablement chauffée à sa température de fusion, ou légèrement supérieure, de préférence entre 65°C et 70 °C.

De préférence de l'agar-agar ou agarose à bas point de fusion est un agar-agar ou agarose dont la température maximale de gélification est comprise entre 24°C et 28°C, et la température de fusion est supérieure à 65,5°C en solution à 1,5 %.

De préférence et comme exemple, mais sans limitation, cet agarose est l'agarose nommé LMP Agarose Low melting point (GIBCOBRL, Life Technologies)

De préférence, ladite deuxième solution est une solution d'agar-agar ou d'agarose à bas point de fusion, dont la concentration en agar-agar ou en agarose à bas point de fusion est comprise entre 1% et 5% (de préférence dans une solution physiologique), de manière plus préférée comprise entre 2% et 5%, entre 3% et 4,5%, entre 3,5% et 4, 5% ou encore entre 3,8% et 4,2% ou entre 3,9% et 4,1%, 4% étant la concentration la plus préférée.

Cette deuxième solution en agar-agar ou en agarose à bas point de fusion à ladite concentration et une fois chauffée à sa température de fusion, ou légèrement supérieure, permet d'être conservée sous forme liquide pendant au moins 1 heure et, de préférence pendant au moins 4heures, 10 heures ou 16 heures à 37 °C.

De préférence, dans ladite matrice liquide comprenant ladite première et ladite deuxième solution d'agar-agar ou d'agarose à bas point de fusion, la concentration finale en agar-agar ou en agarose à bas point de fusion est comprise entre 0,1% et 2%, de préférence entre 0,2% et 1,8%.

Une telle concentration permet d'obtenir non seulement une matrice qui une fois solidifiée permet de conserver la structure 3D et de maintenir en survie ladite biopsie de peau, mais également d'obtenir une matrice solide mais suffisamment souple pour être non cassante et résistante à des chocs ponctuels. La solidification de cette matrice liquide se faisant après dépôt de la biopsie de peau en laissant le dispositif ainsi obtenu à température comprise entre 37 °C et la température ambiante, de préférence à 20 °C.

Selon un mode de réalisation particulier, dans ladite matrice liquide comprenant ladite première et ladite deuxième solution d'agar-agar ou d'agarose à bas point de fusion, la concentration finale en agar-agar ou en agarose à bas point de fusion est comprise entre 1% et 2%, de préférence entre 1, 25% et 1, 75%, de préférence entre 1,4% et 1,6%, 1,5% étant la concentration la plus préférée.

Selon un autre mode de réalisation particulier, dans ladite matrice liquide comprenant ladite première et ladite deuxième solution d'agar-agar ou d'agarose à bas point de fusion, la concentration finale en agar-agar ou en agarose à bas point de fusion est comprise entre 0,1% et 2%, de préférence entre 0, 2% et 1,75%, 0,25% étant la concentration la plus préférée. Une telle concentration permet d'obtenir une matrice qui une fois solidifiée permet de conserver la structure 3D et de maintenir en survie ladite biopsie de peau, mais également d'obtenir une matrice suffisamment souple pour être non cassante et résistante à des effets mécaniques appliqués à la biopsie, par exemple lors d'un effet mimant le massage de la peau pour l'application d'une préparation telle que, par exemple, une crème. La solidification de cette matrice liquide se fait après dépôt de la biopsie de peau en laissant le dispositif ainsi obtenu à température comprise entre 37 ° C et la température ambiante, de préférence à 20 °C.

De préférence, le volume de ladite matrice liquide est de 1/3 à 2/3 du volume total de l'insert, de préférence de 2/5 à 3/5 du volume total, La moitié du volume total de l'insert étant le volume préféré.

De préférence, ladite matrice liquide apte à pouvoir se solidifier contient en outre des cellules autres que les cellules qui composent ladite biopsie de peau, de préférence choisies dans le groupe de cellules constitué par les fibroblastes, des cellules endothéliales ou des cellules nerveuses.

Dans un mode de réalisation encore plus préféré, lesdites cellules sont des fibroblastes, de préférence des fibroblastes primaires (par opposition à des lignéees de fibroblastes) de préférence encore des fibroblastes dermiqueset/ou obtenus à partir de prépuce humain.

Ces fibroblastes primaires, notamment dermiques, peuvent être préparés et obtenus à partir de méthodes standards bien connues de l'homme de l'art (voir par exemple le document Howard BV et al., A new method for the establishment of diploid fibroblast cell cultures from human foreskins. Proc Soc Exp Biol Med. 1976 Nov;153(2):280-3).

De préférence, lesdites cellules, notamment les fibroblastes sont contenues dans la matrice à une concentration entre 5.10³ et 5.10⁵ par ml, de préférence encore entre 10⁴ et 10⁵ par ml, entre 3.10⁴ et 5.10⁴ par ml étant la concentration la plus préférée.

Le procédé selon l'invention tel qu'il est décrit permet à la fois la culture et un transport sécurisé des biopsies de peau (par voie terrestre, maritime ou par les airs). En effet, la biopsie de peau est non seulement emprisonnée et donc maintenue fermement par la matrice solide dans l'insert à membrane poreuse, mais également nourrie, pouvant ainsi voyager sans milieu de culture lors de son transport tout en étant maintenue en survie.

Par culture, on entend désigner ici en particulier la maintenance de l'état physiologique et, le cas échéant, morphologique de l'explant de peau, et donc desdites cellules qui le compose.

Ce procédé a pour avantage de pouvoir effectuer le dépôt de la biopsie de peau dans une première étape sur une phase liquide (au moyen notamment d'un insert contenant ladite matrice sous forme liquide, matrice liquide dont on peut induire la solidification), et, dans une seconde étape, provoquer la solidification de cette matrice liquide, cette solidification permettant de fixer ou figer la structure 3D de ladite biopsie, ceci permettant d'obtenir la conservation ou la maintenance en survie d'un tel explant et son transport possible tout en maintenant également sa structure 3D.

Ce procédé se révèle avantageux par le fait qu'il permet d'obtenir biopsie de peau qui peut être conservée ou maintenue en survie de manière prolongée sans dégradation notable, en outre apte à être transportée, que ce soit par voie terrestre, maritime ou aérienne.

Ladite biopsie de peau comprendra de préférence au moins l'épiderme, le derme et les annexes épidermiques.

De préférence, ledit épiderme comprend l'ensemble de ses couches cellulaires ainsi que les annexes épidermiques dans leur totalité.

Par « ensemble de ses couches cellulaires » pour un épiderme, on entend désigner ici la couche basale, la couche épineuse, la couche granuleuse et la couche cornée. Par « annexes épidermiques dans leur totalité», on entend désigner ici les follicules pileux, les glandes sébacées et les glandes sudoripares.

Cette biopsie de peau pourra comprendre également une partie du tissu sous-cutané appelé encore hypoderme.

De préférence aussi, ladite biopsie de peau est une biopsie de mammifère choisi parmi l'homme ou le porc.

De préférence aussi, ladite biopsie de peau est fraîchement prélevée pour permettre sa survie *in vitro* ou *ex vivo.*

Par fraichement prélevé, on entend ici désigné un prélèvement effectué depuis moins de 1 heure, de préférence moins de 24 heures.

Les biopsies de peau sont préparées de préférence à partir de peau saine, provenant de n'importe quelle localisation du corps du mammifère comme par exemple mais sans s'y limiter de l'abdomen, poitrine, sein (ou mamelles), cuir chevelu, postérieur etc...).

Les biopsies de peau sont préparées de préférence également à partir de peau de mammifère présentant une pathologie, comme par exemple mais sans s'y limiter à partir de tissus pathologiques issus d'exérèse, et provenant par exemple de patient ou animal présentant un cancer cutané (mélanome, cancers baso- ou spino-cellulaires, autre type de cancer cutané), une plaque de psoriasis, lésion d'eczéma ou une dermatite atopique ou tout autre type de pathologie cutanée.

Sous un aspect particulier, ladite biopsie de peau utilisée dans ce procédé est une biopsie de peau issue d'une collection ou obtenue après une culture de peau.

De préférence, les biopsies de peau sont de forme cylindrique.

De préférence, les biopsies de peau sont de forme cylindrique dont le diamètre peut varier entre 1 mm et 50 mm, de manière plus préférée entre 1 et 20 mm de diamètre, de préférence encore entre 1 et 10 mm.

Selon un mode de réalisation également préféré, ladite biopsie préalablement prélevée est une biopsie de forme cylindrique dont l'épaisseur est comprise entre 1 et 20 mm, de préférence entre 2 et 15 mm d'épaisseur, de préférence encore entre 2 et 10 mm.

Dans un mode de réalisation préféré, ledit insert dont le fond est constitué d'une membrane poreuse est un insert en forme de nacelle, de préférence dont le diamètre est compris entre 5 et 40 mm, de manière plus préférée entre 9,5 et 30 mm.

De préférence encore, ledit insert et un insert suspendu ou sur pilotis, de préférence suspendu.

De préférence encore, ladite membrane poreuse est une membrane d'une porosité permettant d'éviter à la matrice liquide apte à pouvoir se solidifier de couler au travers de la membrane avant sa solidification, de préférence encore choisie entre 0,4 à 8 µm, de manière préférée encore entre 0,4 et 1,5 µm, entre 0,8 µm et 1,2 µm étant la porosité préférée.

De préférence encore, ladite membrane poreuse est une membrane choisie en polyéthylène téréphtalate (PET), nitrocellulose ou polycarbonate.

Parmi ces inserts, on peut citer ceux fournis en particulier par la société Nunc (Roskilde, Danemark), BD Falcon (Becton Dickinson France SAS, 38801 Le Pont-De-Claix, France, Millicell® (EMD Millipore Corporation, Billerica, MA, USA) ou Costar® (Grosseron SAS, 44819 Saint-Herblain France), par exemple les inserts avec membrane en polycarbonate, en PET ou nitrocellulose pré-emballés dans des plaques multi-puits pour boîtes de Pétri 6, 8,12 et 24 puits et dont la porosité de la membrane peut varier entre 0,4 et 8µm, les boites de 8 puits et/ou avec une porosité de membrane de 0,8 µm à 1 µm et/ou en PET tétant les plus préférées.

Dans un mode de réalisation préféré, ledit container dans lequel est déposé ledit insert est un puits d'une plaque de culture cellulaire de 6, 8, 12, 24, ou 48 puits. Parmi ces plaques de cultures on peut citer celles fournies en particulier par la société Nunc, BD Falcon, ou sous les références Millicell® ou Costar®.

De préférence, le fond de l'insert est situé à une distance comprise entre 1mm et 2,5 mm du fond du container contenant ledit insert, notamment du fond du puits de la plaque de culture (ou du fond de la boite de Pétri selon l'appellation).

Dans un autre mode de réalisation préféré, à l'étape a), ladite matrice liquide apte à pouvoir se solidifier contient entre 1 mM et 5 mM de Ca²⁺, de préférence entre 1,5 mM et 4,5 mM de Ca²⁺. Selon un mode de réalisation préféré, à l'étape a) ladite matrice liquide apte à pouvoir se solidifier contient entre 1 mM et 2 mM de Ca²⁺, de préférence entre 1,2 mM et 1,4 mM de Ca²⁺. Selon un autre mode de réalisation préféré, à l'étape a) ladite matrice liquide apte à pouvoir se solidifier contient entre 2 mM et 3 mM de Ca²⁺, de préférence entre 2,5 mM et 2,8 mM de Ca²⁺ et plus préférentiellement 2,8 mM de Ca²⁺.

De préférence également, ladite matrice liquide apte à pouvoir se solidifier contient entre 5 et 500 mg/mL d'acide ascorbique, de préférence entre 25 et 75 mg/mL, 50 mg/mL d'acide ascorbique étant la concentration la plus préférée.

De préférence encore, ladite matrice liquide apte à pouvoir se solidifier est un milieu contenant entre 1 mM et 5 mM de Ca²⁺ et contenant entre 5 et 500 mg/mL d'acide ascorbique.

Dans un mode de réalisation préféré, ladite matrice liquide apte à pouvoir se solidifier contenue dans l'insert à l'étape a), est une matrice liquide, dont la première solution du mélange (la deuxième solution étant la solution d'agar-agar ou d'agarose) est de préférence nutritive, et également apte à pouvoir se solidifier sous l'action d'une augmentation ou d'une diminution de la température et/ou par l'addition d'un composé ou d'une composition spécifique.

De préférence ladite matrice liquide apte à pouvoir se solidifier ne contient aucun facteur de croissance ni sérum animal ou humain.

De préférence, à l'étape a), ladite matrice liquide apte à pouvoir se solidifier ne recouvre pas la face supérieure de l'épiderme avant que cette matrice ne soit solidifiée à l'étape b).

Selon un autre mode de réalisation également préféré, à l'étape a) dudit procédé, la biopsie de peau est ensuite placée sur une matrice liquide apte à pouvoir se solidifier, notamment comme ci-dessus indiqué, et laquelle matrice liquide est choisie parmi toute solution liquide apportant tous les ingrédients nutritifs et/ou nécessaires à sa culture, en particulier à la maintenance de l'état de physiologie initiale des cellules qui le constituent. Cette solution est capable de se solidifier ou de se gélifier dans des conditions particulières compatibles avec la survie et la culture de la biopsie de la peau.

De préférence, ladite matrice liquide apte à pouvoir se solidifier est une solution liquide dérivée de plasma sanguin traité avec un agent anticoagulant aux propriétés réversibles mélangée à une solution d'agar-agar ou d'agarose.

De préférence, ladite matrice liquide apte à pouvoir se solidifier contient un plasma sanguin, du fibrinogène, ou du collagène, mélangé à une solution d'agar-agar ou d'agarose à bas point de fusion,

Selon un mode de réalisation également préféré du procédé selon l'invention, à l'étape a), ladite matrice liquide apte à pouvoir se solidifier est une solution dérivée de plasma sanguin contenant de 25 % à 60 %, de préférence entre 35 % et 45 % (v/v) de plasma sanguin, de 70 % à 35 % d'une solution physiologique, telle qu'une solution de NaCl à 0,9 %, de 5 % à 12 %, de préférence 8 %, d'une solution saline de CaCl₂ à 1 %, d'un agent anti-fibrinolytique en concentration suffisante pour obtenir l'activité anti-fribrinolytique recherchée, de préférence entre 5 % et 2 %, de préférence l'agent anti-fibrinolytique étant choisi parmi l'acide tranaxémique ou de l'aprotinine, et d'une solution d'agarose à bas point de fusion entre 0,5% et 4%, de préférence entre 1 % et 2 %.

Selon un mode de réalisation également préféré du procédé selon l'invention, à l'étape a), ladite matrice liquide apte à pouvoir se solidifier est une solution liquide de fibrinogène et de thrombine, ou de collagène ou de plasma sanguin, mélangée à une solution de gélatine, comprenant des gels polymériques synthétiques, naturels tels que les gels d'agarose, en particulier les gels d'agarose ou d'agar-agar à faible/bas points de fusion, les gels d'amidon ou de polysaccharides et pour laquelle l'incubation à 37°C permet sa solidification.

Selon un mode de réalisation également préféré du procédé selon l'invention, à l'étape a) :
- ladite matrice liquide apte à pouvoir se solidifier contient une solution liquide dérivée de plasma sanguin traité avec un agent anticoagulant aux propriétés réversibles, de préférence par du citrate de sodium ; et
- la solidification de ladite matrice à l'étape b) pour cette solution pouvant être obtenue en présence d'ions calcium, de préférence également en présence de thrombine.

Lorsque la matrice liquide apte à pouvoir se solidifier est une matrice liquide contenant une solution de plasma sanguin, une solution de fibrinogène ou de collagène, la solidification de ladite matrice à l'étape b) peut être réalisée pour cette solution par ajout de thrombine ou par augmentation de la température, ou encore à l'aide des facteurs sécrétés par des cellules apportées à la matrice, telles que les fibroblastes primaires.

Selon un mode de réalisation également préféré du procédé selon l'invention, à l'étape b), ladite matrice liquide apte à pouvoir se solidifier est solidifiée après au maximum de 8 heures, de préférence moins de 2 heures ou moins d'une heure, une durée de moins de 30 min, de préférence de moins de 10 min étant la durée la plus préférée pour engager la phase de solidification de la matrice liquide après dépôt de la biopsie de peau sur cette matrice liquide à l'étape a).

Sous un autre aspect, l'invention porte sur l'utilisation d'une telle biopsie de peau obtenu par un tel procédé selon la présente invention à des fins de modèle *ex vivo* ou *in vivo,* en particulier pour des analyses toxicologiques, en particulier des analyses d'absorption percutanée, de métabolisme, de sensibilisation, corrosion ou d'irritation, ou à des fins de recherche, en particulier certains paramètres exogènes comme les UV, le stress, les médicaments ou principe actifs à effet cosmétique, ou encore certaines protéines de signalisation comme les cytokines pourraient être appliquées sur de tels modèles pour étudier les réponses des cellules et des tissus qui composent la peau ou qui y sont associés.

De préférence, lesdites analyses toxicologiques sont choisies parmi les tests de sensibilisation, d'absorption, de métabolisme, de corrosion, ou d'irritation de la peau.

Sous un autre aspect, la présente invention a pour objet un procédé *ex vivo* ou *in vitro* de conservation ou de maintenance en survie d'une biopsie de peau apte à être transportée, ledit procédé comprend les étapes suivantes :
A) la conservation ou maintenance en survie de la biopsie de peau préalablement prélevée et apte à être transportée, directement obtenue par un procédé selon la présente invention;
B) le transport de ladite biopsie de peau ainsi obtenue à l'étape A) ; et
C) la culture de ladite biopsie de peau ainsi obtenue après transport à l'étape B) dans des conditions de cultures adéquates et/ou présence du ou des composés que l'on souhaite tester, l'épiderme de ladite biopsie de peau étant au contact de l'air.

De préférence, à l'étape C), la culture de ladite biopsie de peau ainsi obtenue après transport à l'étape B) dans des conditions de cultures adéquates et/ou présence du ou des composés que l'on souhaite tester, peut être réalisée au moyen de milieu de culture additionnel, pouvant contenir lesdits composés, et ajouté dans le container ou puits, ce milieu additionnel pouvant diffuser au travers de la membrane poreuse de l'insert.

Selon un mode de réalisation également préféré du procédé selon l'invention, à l'étape C), ladite biopsie de peau ainsi disposée est cultivée pendant une période entre 1 et 15 jours et de manière préférentielle entre 1 et 7 jours, et à une température comprise entre 4°C et 37°C.

Selon un autre aspect, l'invention a pour objet l'utilisation d'un dispositif pour la conservation et/ou la maintenance en survie d'une biopsie de peau directement obtenue par le procédé de l'invention comprenant un insert dont le fond est constitué d'une membrane poreuse, ledit insert étant disposé dans un puits, et ledit insert contenant une matrice liquide apte à pouvoir se solidifier sous l'action d'une augmentation ou d'une diminution de la température et/ou par l'addition d'un composé ou d'une composition spécifique, ladite matrice apte à pouvoir se solidifier étant choisie parmi le plasma sanguin ou une solution dérivée de plasma sanguin dilué en tampon physiologique à au maximum 10 %, une solution de fibrinogène, une solution de collagène, de la gélatine, les gels polymériques synthétiques, les gels naturels d'agarose ou d'agar-agar à faible/bas points de fusion, les gels d'amidon ou de polysaccharides, ou un de leurs mélanges.

Selon un autre aspect, l'invention a pour objet un dispositif pour la conservation et/ou la maintenance en survie d'une biopsie de peau comprenant un insert dont le fond est constitué d'une membrane poreuse, ledit insert étant disposé dans un puits, et ledit insert contenant une matrice solidifiée ou gélifiée adhérant aux parois latérales et à ladite membrane poreuse de l'insert.

Dans un dispositif selon l'invention, ledit insert est disposé dans un container ou un puits vide, de type cupule de microplaque ou plaque pouvant servir à la culture cellulaire, de préférence à fond plat. De préférence, ledit insert est suspendu dans le container ou le puits et peut être retiré du container ou du puits.

Dans un mode particulier de réalisation d'un dispositif selon l'invention, ladite matrice liquide apte à pouvoir se solidifier ou ladite matrice solidifiée ou gélifiée adhérant aux parois latérales et à ladite membrane poreuse de l'insert, est choisie parmi toute solution liquide, de préférence nutritive, capable de se solidifier ou se gélifier dans des conditions particulières compatibles avec la survie et la culture de cellules de peau qui composent ladite biopsie, de préférence choisie parmi le plasma sanguin ou une solution dérivée de plasma sanguin, notamment dilué en tampon physiologique à au maximum 10%, de préférence à au moins 20%, au moins 30% et 40%, une solution de fibrinogène, une solution de collagène, de la gélatine, les gels polymériques synthétiques, naturels tels que les gels d'agarose, en particulier les gels d'agarose ou d'agar-agar à faible/bas point de fusion, les gels d'amidon ou de polysaccharides, ou un de leurs mélanges.

De préférence, ladite matrice liquide apte à pouvoir se solidifier comprend une solution dérivée de plasma sanguin, une solution de fibrinogène ou de collagène, une solution d'agar-agar ou d'agarose à bas point de fusion ou un mélange de ces solutions.

De préférence, dans ladite matrice liquide apte à pouvoir se solidifier comprenant ladite première et ladite deuxième solution d'agar-agar ou d'agarose à bas point de fusion, la concentration finale en agar-agar ou en agarose à bas point de fusion est comprise entre 0,1% et 2%, de préférence entre 0,2 et 1,8 %.

Selon un mode de réalisation particulier, dans ladite matrice liquide apte à pouvoir se solidifier comprenant ladite première et ladite deuxième solution d'agar-agar ou d'agarose à bas point de fusion, la concentration finale en agar-agar ou en agarose à bas point de fusion est comprise entre 1% et 2%, de préférence entre 1,25% et 1, 75%, de préférence entre 1, 4% et 1, 6%, 1,5% étant la concentration la plus préférée.

Selon un autre mode de réalisation particulier, dans ladite matrice liquide apte à pouvoir se solidifier comprenant ladite première et ladite deuxième solution d'agar-agar ou d'agarose à bas point de fusion, la concentration finale en agar-agar ou en agarose à bas point de fusion est comprise entre 0,1% et 2%, de préférence entre 0, 2% et 1, 75%, 0,25% étant la concentration la plus préférée.

De préférence, la flottaison d'une biopsie de peau dans une matrice d'un dispositif selon l'invention laisse la surface épidermique émergée tandis que le derme est immergé.

Sous un autre aspect, la présente invention a pour objet une biopsie de peau susceptible d'être obtenue ou directement obtenue par le procédé selon la présente invention, comme modèle.

Selon cet aspect de l'invention, a également pour objet un kit comprenant une telle biopsie de peau comme modèle, ce kit notamment reposant sur la présence d'une matrice solide, cette matrice solide étant adaptée par sa composition à la conservation et/ou la maintenance en survie et de la structure, en particulier 3D, de ladite biopsie de peau et à son transport.

Ainsi, selon cet aspect, l'invention a pour objet également un kit, en particulier pour l'évaluation ou pour la sélection de composé cosmétique, dermatologique ou thérapeutique destiné à être utilisé pour la peau, ledit kit comprenant une telle biopsie de peau comme modèle de peau et obtenue selon le procédé de l'invention.

La présente invention divulgue une méthode de screening *in vitro* de composés candidats pour le traitement cosmétique ou thérapeutique de la peau, cette méthode comprenant les étapes suivantes:
a) l'obtention d'une biopsie de peau comme modèle selon la présente invention;
b) la mise en contact dudit modèle avec le composé candidat ; et
c) la mise en évidence d'une modification physiologique; et
d) la sélection dudit composé si les modifications obtenues sont celles souhaitées pour le traitement.

Un autre objet de la présente invention se rapporte à une méthode permettant de réaliser des prélèvements successifs de couches cellulaires superficielles à l'aide d'adhésifs (méthode de « tape-stripping »), caractérisée en ce que ces prélèvements sont réalisés sur un modèle de biopsie de peau obtenue par le procédé de l'invention. Ces couches cellulaires ainsi obtenues pouvant être analysées de façon indépendante de la biopsie entière.

Ainsi la biopsie de peau comme modèle selon l'invention peut aussi être utilisée dans tout procédé, notamment automatisé, pour le criblage ou l'identification de nouveaux composés cosmétiques, dermatologiques ou pharmaceutiques destinés en particulier à être appliqués sur la peau.

Les procédés de criblage en vue d'identifier de nouveaux composés efficace comprennent en général une de mise en contact dudit composé à tester avec un modèle de peau obtenu selon l'invention puis une étape de lecture de l'effet du composé sur ledit modèle, notamment en comparant cet effet avec un modèle contrôle ou témoin selon l'invention n'ayant pas été mis en contact avec le composé à tester. Cette dernière étape de lecture pourra également être réalisée par la détermination ou l'analyse de marqueurs épidermiques et/ou de cellules associées et incluses dans la matrice solidifiée, tels que des protéines associées à ces structures. Par exemple si ces cellules associées sont des cellules du système immunitaire, ledit procédé de criblage sera par exemple destiné à identifier ou sélectionner des composés tests susceptibles d'induire des effets secondaires non recherchés tels que des réactions allergiques ou de sensibilisation.

Les produits testés pourront également être des vecteurs d'expression de gènes, ou encore des acides nucléiques, tels que des acides nucléiques antisens, microRNA, siRNA aptes à modifier l'expression d'un gène constitutif de cellules présentes dans ledit modèle de l'invention.

D'autres effets tels que l'expression de certains médiateurs ou la cytotoxicité intrinsèque des composés tests vis à vis de certaines cellules de la peau pourront être également recherchés comme effet secondaire, ou vis à vis de certaines cellules associées qui auront pu être insérées en outre avec la biopsie de peau dans la matrice avant sa solidification.

Ces modèles témoins seront bien entendus mis en oeuvre dans les mêmes conditions que le modèle de l'invention ayant reçu le produit à tester.

La présente invention divulgue également un procédé pour déterminer le traitement thérapeutique adapté pour un individu souffrant d'un désordre ou pathologie de la peau, notamment de l'épiderme ou du derme, ce procédé comprenant les étapes suivantes :
a) à partir d'une biopsie de peau prélevée chez ledit patient, l'obtention d'un modèle selon la présente invention;
b) la mise en contact dudit modèle avec un composé candidat pour ledit traitement ;
c) la mise en évidence de modification physiologique ou morphogénique de la peau, notamment de l'épiderme ou du derme, associée à l'efficacité du traitement des pour ledit désordre ou ladite pathologie; et
d) la sélection dudit composé si les modifications obtenues sont celles souhaitées pour le traitement.

La présente invention divulgue un procédé pour déterminer le traitement thérapeutique adapté pour un individu souffrant d'un désordre de la peau, notamment de l'épiderme ou du derme, caractérisé en ce qu'à l'étape a), la biopsie de peau utilisée est issue d'une collection ou de culture de peau.

Sous un autre aspect, la présente invention a pour objet l'utilisation d'un modèle selon la présente invention, pour déterminer pour un produit les effets secondaires sur la peau tels que par exemple :
- sa toxicité ;
- son adsorption ou absorption ;
- sa distribution, son métabolisme, son excrétion ;
- son pouvoir irritant ou corrosif;
- son pouvoir sensibilisant.

Un tel modèle pourra être en effet selon la présente invention être utilisé ou mis en oeuvre dans tout procédé ou test *in vitro* ou *ex vivo* nécessitant des expérimentations animales ou humaines, comme par exemple l'étude de libération ou de pénétration de principes actifs, et/ou de leur biodisponibilité cutanée, l'étude de leur efficacité, ou encore de leur tolérance, de leur compatibilité, ces principes actifs étant à visée cosmétiques, dermatologiques et/ou pharmaceutiques.

Selon un mode de réalisation préféré, ledit produit testé est un produit cosmétique, dermatologique ou pharmaceutique.

L'utilisation comme modèle concernera également sa mise en oeuvre pour l'étude de toute pathologie se traduisant par des anomalies de la peau, notamment de l'épiderme et/ou du derme, en particulier également pour toutes les cellules situées sous l'épiderme pour lesquelles l'épiderme constitue une barrière naturelle et pour lesquelles une thérapie par voie topique est envisageable.

D'autres caractéristiques et avantages de l'invention apparaissent dans la suite de la description avec l'exemple et les figures. Les figures dont les légendes sont ci-après, ainsi que les exemples qui suivent sont destinés à illustrer l'invention sans pour autant en limiter la portée.

### LEGENDES DES FIGURES

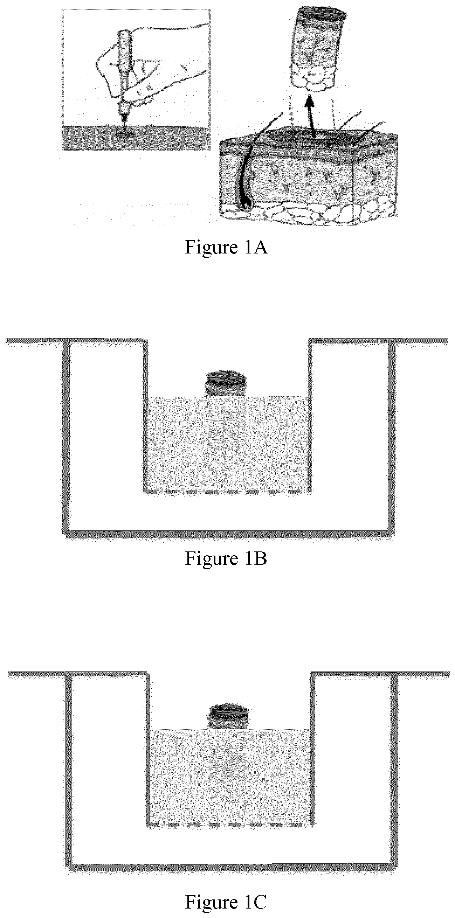

**Figures 1A, 1B et 1C** : Schéma décrivant le procédé selon l'invention
- Etape 1 : Une biopsie cylindrique de peau est réalisée (Figure 1A);
- Etape 2 : La biopsie est placée en flottaison dans un container/insert à membrane poreuse contenant une solution préparée avec un mélange de plasma sanguin et d'agarose fondu, ledit insert étant lui-même disposé (suspendu) dans un puits de plaque de culture cellulaire non pré-rempli de milieu nutritif (Figure 1B);
- Etape 3 : La solution de plasma sanguin est solidifiée par coagulation; et la biopsie disposée sur le plasma coagulé est maintenu en culture grâce à la diffusion du milieu de culture au travers de la membrane poreuse de l'insert (Figure 1C).

**Figure 2** **:** Schéma décrivant la fixation de l'anneau hydrophobe sur la surface épidermique de la biopsie.

**Figures 3A et 3B** **:** Schéma représentant la biopsie associée à l'anneau, placée en flottaison dans un insert à membrane poreuse contenant la matrice, dans lequel le puits de culture cellulaire n'est pas pré-rempli de milieu nutritif (Figure 3A), ou dans lequel le puits de culture cellulaire est rempli de milieu nutritif (Figure 3B).

### EXEMPLES

### EXEMPLE 1 : Mise en oeuvre du procédé (voir Figures 1A, 1B et 1C)

Un dispositif pour la conservation et/ou la maintenance en survie d'une biopsie de peau comprend un insert dont le fond est constitué d'une membrane poreuse. L'insert contient une matrice liquide apte à se solidifier ou à se gélifier, l'insert est disposé dans un puits.
1. Une biopsie de peau cylindrique (diamètre 8 mm, épaisseur 5 mm) est tout d'abord réalisée sur un fragment de peau fraîchement prélevé pour faciliter sa survie *ex vivo* ou *in vitro.*
2. La biopsie est délicatement déposée sur un insert (cupule Millicell™ 8 puits) disposant au fond d'une membrane poreuse (en PET, porosité 1 µm), cet insert contenant une solution dérivée de plasma sanguin traité avec un agent anticoagulant aux propriétés réversibles en présence d'ions calcium (citrate de sodium). Cette solution contient 42 % de plasma sanguin, 50 % d'une solution de NaCl à 0,9 %, 8 % d'une solution saline de CaCl2 à 1%, un agent anti-fibrinolytique (acide tranaxémique ou aprotinine), et de l'agarose fondue à bas point de fusion à 1,5% (Agarose LMP GIBCOBRL, Life Technologies) (fondu à l'étuve à 65,5 °C).
3. En coagulant, le plasma fait office de support dermique sur lequel adhère l'explant de peau. La coagulation consiste essentiellement en la transformation, en présence d'ions calcium et de thrombine, du fibrinogène présent dans le plasma en un échafaudage de molécules de fibrine reliées entre elles par des liaisons covalentes. L'agent anti-fibrinolytique a pour fonction d'inhiber les enzymes susceptibles de dégrader la matrice de plasma, ces enzymes étant sécrétées par l'explant de peau, et ainsi de maintenir l'intégrité de l'explant. De la même façon la solution de plasma peut être substituée par une solution de fibrinogène ou de collagène. Pour cette dernière, l'incubation à 37°C seule permet la solidification.
4. La solution d'agarose à 1,5% contenue dans la solution de plasma gélifie progressivement à 37°C permettant ainsi le maintien ferme de la biopsie de peau dans l'insert. La biopsie de peau peut être ainsi transportée sans être altérée pendant une durée de 24h, 48h ou davantage, selon les besoins.
5. L'ensemble constitué par l'explant de peau (ou encore appelé ici fragment ou biopsie de peau) est maintenu en culture avec l'épiderme au contact de l'air et le milieu nutritif contenu dans l'insert disposé (suspendu) sur le puits de la plaque de culture cellulaire. En cas de besoin, l'insert mobile peut être soulevé, permettant ainsi l'ajout ou le renouvellement de milieu nutritif dans le puits, ou l'ajout d'un additif particulier au milieu nutritif. Selon un mode de réalisation particulier, ledit additif est un composé destiné à être testé lors de son contact avec le fragment ou la biopsie.

La présence d'une membrane poreuse dans le fond de l'insert permet la diffusion des éléments nutritifs à travers la membrane, et le cas échéant la diffusion de composés produits par le fragment ou la biopsie de peau lors de la culture et dont l'accumulation locale pourrait être dommageable pour ledit fragment ou biopsie.

### EXEMPLE 2: Mise en oeuvre du procédé dans lequel un anneau hydrophobe est fixé à la biopsie

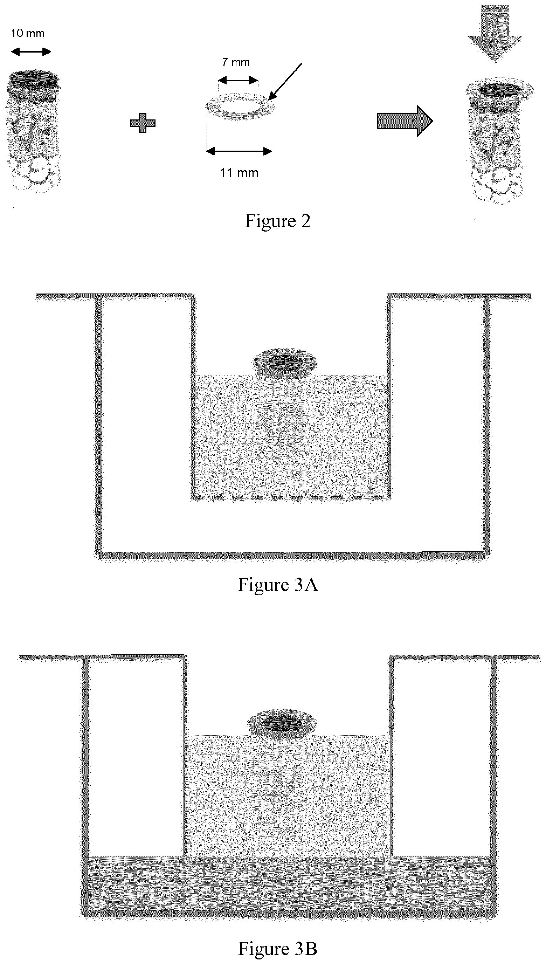

Un anneau, ou disque perforé, dont le diamètre intérieur est de 7 mm et le diamètre extérieur est de 11 mm est découpé dans un film hydrophobe de type Parafilm ® (Sigma) dont l'épaisseur comprise entre 100 et 120 Microns. Une biopsie de peau cylindrique de diamètre 10 mm est réalisée sur un fragment de peau fraîchement prélevé pour faciliter sa survie ex vivo ou in vitro. La face inférieure de l'anneau est enduite de silicone liquide, qui permet l'adhésion de l'anneau à la biopsie. L'anneau est disposé par pression sur la surface épidermique de la biopsie, de façon à ce qu'il chevauche la surface de la biopsie (Figure 2). Le disque est disposé de façon à ce que son centre corresponde à celui de la biopsie. L'ensemble constitué par la biopsie et l'anneau hydrophobe est délicatement déposé sur un insert (Millicell™ hanging inserts, Merck Millipore) disposant au fond d'une membrane poreuse (en PET, porosité 1 µm), cet insert contenant une solution dérivée de plasma sanguin telle que décrite dans l'exemple 1. Dans ce cas, l'incubation à 37°C seule permet la solidification, la solution d'agarose à 1,5% contenue dans la solution de plasma gélifie progressivement à 37°C permettant ainsi le maintien ferme de la biopsie de peau dans l'insert (Figure 3A). L'ensemble constitué par la biopsie et l'anneau hydrophobe est maintenu en culture, avec l'épiderme au contact de l'air, au contact du le milieu nutritif contenu dans le puits de la plaque de culture cellulaire dans lequel l'insert est disposé (Figure 3B).

Selon un exemple particulier, la présence d'un anneau hydrophobe délimitant une zone de 0,5 cm2 permet de déposer et de maintenir en contact avec la biopsie un volume de solution liquide de 50 microlitres.

L'ajout du disque perforé hydrophobe fixé sur la biopsie entraîne une meilleure flottaison de la biopsie sur la matrice liquide (lors du dépôt dans l'insert), la ligne de flottaison passant sous le disque. La présence du disque permet également de délimiter précisément la zone de la surface de la biopsie qui est au contact avec l'air. Cette zone est utilisée pour réaliser des applications topiques. La présence du disque perforé permet également d'éviter la diffusion latérale de solutions ou de formulations qui sont appliquées sur la biopsie, le disque faisant office de barrière à la diffusion. Le disque permet enfin d'éviter le contact de la zone émergée de la surface épidermique de la biopsie avec le mélange de solutions, constituant la matrice lorsque celle-ci est liquide.

### EXEMPLE 3: Mise en oeuvre du procédé avec différentes concentrations finales d'agarose à bas point de fusion

Les propriétés physico chimiques de fragments de peau maintenus dans différentes solutions de matrices ont été comparées.

Une matrice liquide est préparée à partir d'une première solution contenant du plasma, et d'une seconde solution contenant de l'agarose à bas point de fusion, dont la température maximale de gélification est comprise entre 24°C et 28°C et dont la température de fusion est supérieure à 65,5°C.

Dans l'exemple où la proportion finale d'agarose à bas point de fusion dans la matrice (après mise en contact de la première et de la deuxième solution) est de 0,25 %, la consistance de la matrice est adaptée à un bon maintien de la biopsie lors d'un transport routier et aérien, sans altération de la biopsie.

De plus, une matrice solidifiée comprenant 0,25 % d'agarose à bas point de fusion conserve une capacité de déformation appropriée pour le maintien de la biopsie lors du transport, tout en conservant les propriétés d'adhésion de la matrice à la partie immergée de la biopsie. Les propriétés de maintien et de souplesse de la matrice ainsi préparée sont adaptées à la réalisation de tests comportant un effet mécanique sur la biopsie, comme par exemple un effet mimant un massage lors de l'application d'une préparation telle qu'une crème sur la peau. En effet, dans ce cas la biopsie reste fixée dans la matrice solidifiée malgré les contraintes mécaniques qui peuvent lui être appliquées.

## Revendications

1. Procédé *ex vivo* ou *in vitro* pour la maintenance en survie *in vitro* ou *ex vivo* et le transport de biopsie de peau, ladite biospie de peau comprenant au moins l'épiderme, le derme et les annexes épidermiques, ladite biopsie ayant été préalablement prélevée chez un mammifère, ladite méthode comprenant les étapes suivantes :
a) Le dépôt de ladite biopsie de peau sur une matrice liquide apte à pouvoir se solidifier sous l'action d'une augmentation ou d'une diminution de la température et/ou par l'addition d'un composé ou d'une composition spécifique de manière à ce que la partie superficielle de ladite biopsie correspondant à la totalité de l'épiderme reste émergée alors que le derme sous-jacent à cet épiderme est immergé totalement, ladite matrice liquide apte à pouvoir se solidifier étant choisie parmi le plasma sanguin ou une solution dérivée de plasma sanguin dilué en tampon physiologique à au maximum 10 %, une solution de fibrinogène, une solution de collagène, de la gélatine, les gels polymériques synthétiques, les gels naturels d'agarose ou d'agar-agar à faible/bas points de fusion, les gels d'amidon ou de polysaccharides, ou un de leurs mélanges,
ladite matrice liquide apte à pouvoir se solidifier étant elle-même contenue dans un insert dont le fond est constitué d'une membrane poreuse, et
ledit insert étant disposé dans un container ou puit; et
b) La solidification de ladite matrice liquide apte à pouvoir se solidifier induite par une augmentation ou une diminution de la température et/ou par l'addition d'un composé ou d'une composition spécifique, cette solidification ayant pour effet d'emprisonner la partie immergée de ladite biopsie de peau dans ladite matrice ainsi solidifiée, et de faire adhérer ladite matrice solidifiée aux parois latérales et à ladite membrane poreuse de l'insert, et ladite partie superficielle épidermique de la biopsie, restant émergée, étant en contact avec l'air atmosphérique ou sous une atmosphère contrôlée comprenant en partie de l'air.

2. Procédé *ex vivo* ou *in vitro* selon la revendication 1, **caractérisé en ce que** ledit procédé comprend, préalablement à l'étape a) selon la revendication 1, une étape de fixation à la surface épidermique de la biopsie de peau d'un anneau constitué par un matériau hydrophobe, le diamètre extérieur dudit anneau étant supérieur au diamètre de la surface épidermique de la biopsie, le diamètre intérieur dudit anneau étant inférieur au diamètre de la surface épidermique de la biopsie.

3. Procédé selon l'une des revendications 1 ou 2, **caractérisé en ce que** ladite membrane poreuse est une membrane d'une porosité choisie entre 0,4 à 8 µm.

4. Procédé selon l'une des revendications 1 à 3, **caractérisé en ce que** ladite matrice liquide apte à pouvoir se solidifier est un milieu contenant entre 1 mM et 5 mM de Ca²⁺ et contenant entre 5 et 500 mg/mL d'acide ascorbique.

5. Procédé selon l'une des revendications 1 à 4, **caractérisé en ce que** ladite matrice liquide apte à pouvoir se solidifier est une solution liquide dérivée de plasma sanguin traité avec un agent anticoagulant aux propriétés réversibles mélangée à une solution d'agar-agar ou d'agarose.

6. Procédé selon la revendication 5, **caractérisé en ce que** ladite solution liquide dérivée de plasma est une solution liquide dérivée de plasma sanguin contenant de 25 % à 60 % de plasma sanguin, de 70 % à 35 % d'une solution physiologique, de 5 % à 12 %, d'une solution saline de CaCl₂ à 1 % et, le cas échéant, d'un agent anti-fibrinolytique.

7. Procédé selon l'une des revendications 1 à 6, **caractérisé en ce que** ladite matrice liquide apte à pouvoir se solidifier comprend de l'agarose ou de l'agar-agar à faible/bas point de fusion à une concentration finale comprise entre 0,1 % et 5 %.

8. Procédé selon l'une des revendications 1 à 7, **caractérisé en ce que** ladite matrice liquide apte à pouvoir se solidifier contenue dans l'insert ne comprend aucun facteur de croissance ni de sérum animal ou humain.

9. Procédé *ex vivo* ou *in vitro* de conservation ou de maintenance en survie d'une biopsie de peau apte à être transportée, ledit procédé comprenant les étapes suivantes :
A) la conservation ou maintenance en survie de la biopsie de peau préalablement prélevée et apte à être transportée directement obtenue par un procédé selon l'une des revendications 1 à 8;
B) le transport de ladite biopsie de peau ainsi obtenue à l'étape A) ; et
C) la culture de ladite biopsie de peau ainsi obtenue après transport à l'étape B) dans des conditions de cultures adéquates et/ou en présence du ou des composés que l'on souhaite tester, l'épiderme de ladite biopsie de peau étant au contact de l'air.

10. Utilisation d'un dispositif pour la conservation et/ou la maintenance en survie d'une biopsie de peau directement obtenue par un procédé selon l'une des revendications 1 à 8 ou selon la revendication 9, ledit dispositif comprenant un insert dont le fond est constitué d'une membrane poreuse, ledit insert étant disposé dans un puits, ledit insert contenant une matrice liquide apte à pouvoir se solidifier sous l'action d'une augmentation ou d'une diminution de la température et/ou par l'addition d'un composé ou d'une composition spécifique, ladite matrice liquide apte à pouvoir se solidifier étant choisie parmi le plasma sanguin ou une solution dérivée de plasma sanguin dilué en tampon physiologique à au maximum 10 %, une solution de fibrinogène, une solution de collagène, de la gélatine, les gels polymériques synthétiques, les gels naturels d'agarose ou d'agar-agar à faible/bas points de fusion, les gels d'amidon ou de polysaccharides, ou un de leurs mélanges.

11. Kit comprenant une biopsie de peau obtenue par un procédé selon l'une des revendications 1 à 8, pour l'évaluation d'un composé cosmétique, dermatologique ou thérapeutique destiné à être utilisé pour la peau.

## Patentansprüche

1. *Ex-vivo-* oder *in*-*vitro*-Verfahren zur Lebenderhaltung *in vitro* oder *ex vivo* und Transport von Hautbiopsieproben, wobei die genannte Hautbiopsieprobe mindestens die Epidermis, die Dermis und die Epidermis-Anhangsgebilde umfasst, wobei die genannte Biopsieprobe vorher bei einem Säugetier abgenommen wurde, wobei das genannte Verfahren die folgenden Schritte umfasst:
a) Auflegen der genannten Biopsieprobe auf eine flüssige Matrix, die fähig ist, sich unter der Wirkung einer Erhöhung oder Verringerung der Temperatur und/oder durch Zusatz einer spezifischen Verbindung oder Zubereitung zu verfestigen, derart dass der Oberflächenteil der genannten Biopsieprobe, die der gesamten Epidermis entspricht, an der Oberfläche bleibt, während die unter dieser Epidermis liegende Dermis vollständig eingetaucht ist, wobei die genannte flüssige, zur Verfestigung fähige Matrix unter Blutplasma oder einer zu maximal 10% mit physiologischem Puffer verdünnten Blutplasmalösung, einer Fibrinogenlösung, einer Kollagenlösung, Gelatine, synthetischen polymeren Gelen, natürlichen Agarose- oder Agar-Agar-Gelen mit geringem/niedrigem Schmelzpunkt, Stärke- oder Polysaccharidgelen oder einem Gemisch davon ausgewählt wurde,
wobei sich die genannte flüssige, zur Verfestigung fähige Matrix ihrerseits in einem Insert befindet, dessen Boden aus einer porösen Membran besteht, und
das genannte Insert in einem Behälter oder einer Vertiefung angeordnet ist, und
b) Verfestigung der genannten flüssigen, zur Verfestigung fähigen Matrix, hervorgerufen durch eine Erhöhung oder Verringerung der Temperatur und/oder durch Zusatz einer spezifischen Verbindung oder Zubereitung, wobei diese Verfestigung zur Folge hat, den eingetauchten Teil der genannten Hautbiopsieprobe in der genannten so verfestigten Matrix einzuschließen und die genannte verfestigte Matrix an den Seitenwänden und an der genannten porösen Membran des Insertes haften zu lassen, und wobei der genannte oberflächliche, epidermale Teil der Biopsieprobe, der an der Oberfläche bleibt, mit der atmosphärischen Luft in Kontakt bleibt oder mit einer kontrollierten Atmosphäre, die teilweise Luft enthält.

2. *Ex-vivo-* oder *in*-*vitro*-Verfahren nach Patentanspruch 1, **dadurch gekennzeichnet, dass** das genannte Verfahren vor dem Schritt a) von Patentanspruch 1 einen Schritt zur Befestigung eines Ringes aus einem hydrophoben Werkstoff an der epidermalen Oberfläche der Hautbiopsieprobe umfasst, wobei der Außendurchmesser des genannten Ringes größer ist, als der Durchmesser der epidermalen Oberfläche der Biopsieprobe, während der Innendurchmesser des genannten Ringes kleiner ist, als der Durchmesser der epidermalen Oberfläche der Biopsieprobe.

3. Verfahren nach einem der Patentansprüche 1 oder 2, **dadurch gekennzeichnet, dass** die genannte poröse Membran eine Membran mit einer zwischen 0,4 und 8 µm gewählten Porosität ist.

4. Verfahren nach einem der Patentansprüche 1 bis 3, **dadurch gekennzeichnet, dass** die genannte flüssige, zur Verfestigung fähige Matrix ein Medium ist, das zwischen 1 mM und 5 mM Ca²⁺ enthält und zwischen 5 und 500 mg/ml Ascorbinsäure.

5. Verfahren nach einem der Patentansprüche 1 bis 4, **dadurch gekennzeichnet, dass** die genannte flüssige, zur Verfestigung fähige Matrix eine flüssige Lösung ist, die von mit einem Antikoagulans mit reversiblen Eigenschaften behandeltem Blutplasma abgeleitet wurde, gemischt mit einer Agar-Agar- oder Agarose-Lösung.

6. Verfahren nach Patentanspruch 5, **dadurch gekennzeichnet, dass** die flüssige, von Plasma abgeleitete Lösung eine flüssige Lösung ist, die von Blutplasma abgeleitet wurde, die 25% bis 60% Blutplasma enthält, 70% bis 35% einer physiologischen Lösung, 5% bis 12% einer Salzlösung von CaCl₂ zu 1% und gegebenenfalls ein antifibrinolytisches Mittel.

7. Verfahren nach einem der Patentansprüche 1 bis 6, **dadurch gekennzeichnet, dass** die genannte flüssige, zur Verfestigung fähige Matrix Agarose oder Agar-Agar mit geringem/niedrigem Schmelzpunkt in einer Endkonzentration zwischen 0,1% und 5% enthält.

8. Verfahren nach einem der Patentansprüche 1 bis 7, **dadurch gekennzeichnet, dass** die genannte flüssige, zur Verfestigung fähige Matrix, die im Insert enthalten ist, keinerlei Wachstumsfaktor, noch auch tierisches oder menschliches Serum enthält.

9. *Ex-vivo-* oder in-vitro-Verfahren zur Konservierung oder Lebenderhaltung einer transportfähigen Hautbiopsieprobe, wobei das genannte Verfahren die folgenden Schritte umfasst:
A) Konservierung oder Lebenderhaltung der vorher abgenommenen und transportfähigen Hautbiopsieprobe, die unmittelbar nach einem Verfahren nach einem der Patentansprüche 1 bis 8 erhalten wurde,
B) Transport der genannten, so in Schritt A) erhaltenen Hautbiopsieprobe, und
C) Kultur der genannten, so erhaltenen Hautbiopsieprobe nach Transport in Schritt B) unter geeigneten Kulturbedingungen und/oder in Gegenwart der Verbindung/en, die getestet werden soll/en, wobei sich die Epidermis der genannten Biopsieprobe mit Luft in Kontakt befindet.

10. Gebrauch einer Vorrichtung zur Konservierung oder Lebenderhaltung einer Hautbiopsieprobe, die unmittelbar nach einem Verfahren nach einem der Patentansprüche 1 bis 8 oder nach Patentanspruch 9 erhalten wurde, wobei die genannte Vorrichtung ein Insert umfasst, dessen Boden aus einer porösen Membran besteht, wobei das genannte Insert in einer Vertiefung angeordnet ist und eine flüssige Matrix enthält, die fähig ist, sich unter der Wirkung einer Erhöhung oder Verringerung der Temperatur und/oder durch Zusatz einer spezifischen Verbindung oder Zubereitung zu verfestigen, wobei die genannte flüssige, zur Verfestigung fähige Matrix unter Blutplasma oder einer zu maximal 10% mit physiologischem Puffer verdünnten Blutplasmalösung, einer Fibrinogenlösung, einer Kollagenlösung, Gelatine, synthetischen polymeren Gelen, natürlichen Agarose- oder Agar-Agar-Gelen mit geringem/niedrigem Schmelzpunkt, Stärke- oder Polysaccharidgelen oder einem Gemisch davon ausgewählt wurde.

11. Kit, eine Hautbiopsieprobe umfassend, die nach einem Verfahren nach einem der Patentansprüche 1 bis 8 erhalten wurde, zur Bewertung einer kosmetischen, dermatologischen oder therapeutischen Zubereitung, die dazu bestimmt ist, für Haut verwendet zu werden.

## Claims

1. An *ex vivo* or *in vitro* method for *in vitro* or *ex vivo* keeping alive and for transporting of a skin biopsy, said skin biopsy comprising at least the epidermis, dermis and epidermal appendices, said biopsy having previously been obtained from a mammalian, said method comprising the following steps:
a) Laying said skin biopsy on a liquid matrix capable of solidifying under the action of an increase or decrease in temperature and/or by the addition of a specific compound or composition so that the superficial part of said biopsy corresponding to the entire epidermis remains emerged while the dermis underlying this epidermis is completely immersed, said liquid matrix capable of solidifying being chosen from blood plasma or a solution derived from blood plasma diluted in physiological buffer at a maximum of 10%, a fibrinogen solution, a collagen solution, gelatin, synthetic polymeric gels, natural agarose or agar-agar gels with low melting points, starch or polysaccharide gels, or a mixture thereof,
said liquid matrix capable of solidifying being itself contained in an insert whose bottom is composed of a porous membrane, and
said insert being disposed in a container or well; and
b) Solidifying said liquid matrix capable of solidifying induced by an increase or decrease in temperature and/or by the addition of a specific compound or composition, this solidification having the effect of trapping the immersed part of said skin biopsy in said matrix thus solidified, and to cause the solidified matrix to adhere to the lateral walls and to the porous membrane of the insert, and wherein said epidermal superficial part of the biopsy, remaining emerged is in contact with the atmospheric air or under a controlled atmosphere partially comprising air.

2. An *ex vivo* or *in vitro* method according to claim 1, **characterized in that** said method comprises, prior to step a) according to claim 1, a step of attaching to the epidermal surface of the skin biopsy a ring made of a water-repellent material, wherein the outer diameter of said ring is greater than the diameter of the epidermal surface of the biopsy, and wherein the inner diameter of said ring is smaller than the diameter of the epidermal surface of the biopsy.

3. A process according to one of claims 1 or 2, **characterized in that** said porous membrane has a porosity selected between 0.4 to 8 µm.

4. A process according to one of claims 1 to 3, **characterized in that** said liquid matrix capable of solidifying is a medium containing between 1 mM and 5 mM Ca2+ and containing between 5 and 500 mg/mL ascorbic acid.

5. A process according to one of claims 1 to 4, **characterized in that** said liquid matrix capable of solidifying is a liquid solution derived from blood plasma treated with an anticoagulant agent having reversible properties mixed with a solution of agar-agar or agarose.

6. A process according to claim 5, **characterized in that** said plasma-derived liquid solution is a liquid solution derived from blood plasma containing from 25% to 60% blood plasma, from 70% to 35% of a physiological solution, from 5% to 12% of a saline solution of CaCl₂ at 1% and, optionally, an anti-fibrinolytic agent.

7. A process according to one of claims 1 to 6, **characterized in that** said liquid matrix capable of solidifying comprises low/melting agarose or agar-agar at a final concentration between 0.1% and 5%.

8. A process according to one of claims 1 to 7, **characterized in that** said liquid matrix capable of solidifying contained in the insert does not include any growth factor nor animal or human serum.

9. An *ex vivo* or *in vitro* method for the conservation or keeping alive of a transportable skin biopsy, said method comprising the following steps:
A) preserving or keeping alive the skin biopsy previously collected and capable of being transported directly obtained by a process according to any of claims 1 to 8;
B) transporting said skin biopsy obtained in step A); and
C) culturing said skin biopsy obtained after transport to step B) under suitable culture conditions and/or in the presence of the compound(s) to be tested, the epidermis of said skin biopsy being in contact with air.

10. Use of a device for the preservation and/or keeping alive of a skin biopsy directly obtained by a process according to one of claims 1 to 8 or according to claim 9, said device comprising an insert whose bottom is composed of a porous membrane, said insert being disposed in a well, said insert containing a liquid matrix capable of solidifying under the action of an increase or decrease in temperature and/or by the addition of a compound or a specific composition, said liquid matrix capable of solidifying being chosen from blood plasma or a solution derived from blood plasma diluted in physiological buffer at a maximum of 10%, a fibrinogen solution, a collagen solution, gelatin, synthetic polymeric gels, natural agarose or agar-agar gels with low melting points, starch or polysaccharide gels, or a mixture thereof.

11. A kit comprising a skin biopsy obtained by a process according to one of claims 1 to 8, for the evaluation of a cosmetic, dermatological or therapeutic compound for use on the skin.
